# EUROPEAN PATENT APPLICATION

(11) **EP 2 280 064 A2**
(43) Date of publication of application: **02.02.2011**
(21) Application number: 09754884.6
(22) Date of filing: 19.03.2009
(51) Int. Cl.: C12N 5/06

(54) **COMPOSITION COMPRISING VEGETABLE PEPTONE FOR PROMOTING STEM CELL PROLIFERATION**

(30) Priority: 01.04.2008 KR 20080030237
(71) Applicant: Biospectrum, Inc., Gyeonggi-do 435-776 (KR)
(72) Inventor: PARK, Deok Hoon, Seongnam-si Gyeonggi-do 463-727 (KR); LEE, Jienny, Seongnam-si Gyeonggi-do 463-070 (KR); LEE, Jong Sung, Anyang-si Gyeonggi-do 430-710 (KR)
(74) Representative: Winkler, Andreas Fritz Ernst
(86) International application number: PCT/KR2009/001415
(87) International publication number: WO 2009/145419

(57) **Abstract**

The present invention relates to a composition for promoting stem cell proliferation which contains a vegetable peptone. More specifically, this invention relates to: a serum-free composition for culturing stem cells which contains a vegetable peptone; a composition for improving skin condition containing the vegetable peptone as an active ingredient; and a composition for improving skin condition containing a culture medium as an active ingredient in which cultured stem cells are removed after being cultured in a serum-free composition medium for stem cell culture containing the vegetable peptone. Since the disclosed serum-free composition for culturing stem cells does not need the use of expensive animal serum, the manufacturing cost can be remarkably lowered. Also the use of anima serum in the medium can basically prevent contamination material caused by the use of animal serum. In addition, the disclosed composition containing the vegetable peptone and the medium in which the stem cells are cultured in the serum-free medium both serve to promote and activated the proliferation of stem cells, and improve various skin conditions.

## Description

### FIELD OF THE INVENTION

The present invention relates to a composition for promoting stem cell proliferation which contains a plant peptone. More specifically, this invention relates to a serum-free composition for culturing stem cells which contains a plant peptone; a composition for improving skin condition containing the plant peptone as an active ingredient; and a composition for improving skin condition containing a culture medium as an active ingredient in which cultured stem cells are removed after being cultured in a serum-free medium for stem cell culture containing plant peptide.

### BACKGROUND OF THE INVENTION

Animal cell industrialization technology, one of the production technology in biomedicine, is in the spotlight as a technology for producing high value biomedicinal agent by mass producing genetically modified human and animal cell cultures. Recent development in gene therapy and cell therapy techniques emphasized the importance of the industrialization technology. Generally, the developmental process undertaken with animal cells derived for medicinal agents, focuses on the safety of the developed product and reducing the high production cost of cell production. The animal cell culture method used until recently requires the presence of serum, such as fetal bovine serum (FBS) or fetal calf serum (FCS) in the cell culture medium. The function of these additives is not fully understood but they are known to increase cell growth, provide nutrition and protect the cells from oxidation or toxins. These additives are essential, where there is no efficient cell growth when depleted. However, expensive serum prices leads to an increase in culture medium prices. Due to the complex components of the serum, the purification of the recombinant proteins is difficult and can raise problems in consistency of test results because of the varying composition between batches during the development process. Since serum is derived from animal sources, high risk of contamination with viruses or infectious prions exist during cell culturing. Even though there are several safety measures used in animal cell culture, prions in bovine serum can still be transferred into the human body suggesting the potential for infection. There are several disadvantages of using conventional serum: i) unpredictability in results when inducing cell growth because of the varying composition of the serum; ii) the possible contamination of serum with infectious material when preparing cell product for human use; iii) detection of bacteriophage and bovine virus in commercially manufactured FBS or FCS; iv) the possible presence of cell culture inhibiting factors such as toxins from bacterial infection; v) the presence of bacterial toxin before filtration; vi) culture medium being a source for biological contamination from microplasmas, bacteriophages and viruses; vii) presence of toxins in culture medium by cross reaction between antibodies and bacteria with gamma-globulin fraction; viii) heat inactivation can reduce the cytotoxin reaction of the immunoglobulin but also destroys unstable reagents therefore giving unreliable results compared to non-heat treated serum; ix) cell surface modification by adsorption/reaction of the serum proteins; x) interference with the purification process of the desired cell product; xi) overgrowth of fibroblast in primary culture.

Since the report in 1950s that artificial medium can be partially substituted by natural medium, several attempts have been made to culture cells free of serum. Currently, in some cases, cells are cultured in serum-free media for a specific purpose. However, it has been reported that these serum-free medium are only suitable for growing certain cell types. The use of protein cell growth factors have been considered but they are too expensive and the growth and product synthesis is less stable compared to serum medium. In addition, it has been reported that serum-free medium cannot be applied for growing stem cells. The most important field for commercializing stem cells is in the area of cell therapy. This is accomplished by using a treatment technique of culturing human stem cells in a test tube and injecting them back in to patient's body. This area of research requires stem cellsdd cultured in a medium that contains minimal amounts of animal medium or serum. When stem cell transplantation is used for treating disease, the risk of infection can be overcome by the benefit of disease treatment, but when stem cell transplantation is used for cosmetic purpose, the risk of being exposed to an infection would be greater than the benefits of stem cell transplant. Therefore, finding an animal serum substitute in the area of stem cell culture has a commercially significant meaning.

It is known that stem cells in the skin are responsible for many of the functions that are directly related to the health of the skin. Skin stem cells are the most important cell groups related in skin health. Stem cells are known to proliferate continuously by cell division and to secrete various cell activators to activate neighboring cells and prevent aging and aid in repair of damaged regions within the cell. Like other cell groups, stem cells lose these abilities as the cells get old. That is, the activity of the stem cells in the skin reduces with aging, cell division decreases and gradually the cells become inactivated, therefore leading to the appearance of age related skin condition such as wrinkles, age spot, slow wound healing and repair.

Throughout this application, various patents and publications are referenced and citations are provided in parentheses. The disclosure of these patents and publications in their entities are hereby incorporated by references into this application in order to more fully describe this invention and the state of the art to which this invention pertains.

### DETAILED DESCRIPTION OF THIS INVETNION

### Technical Problems to be Solved

The present inventors have conducted intensive researches to find a material that can induce stem cell proliferation in a serum-free medium that can replace animal serum. As a result, the inventors have discovered that plant peptone was effective enough to be substitute with the animal serum for inducing stem cell proliferation, and thus completed the present invention.

Accordingly, it is an object of this invention to provide a serum-free medium composition for culturing a stem cell comprising a plant peptone.

It is another object of this invention to provide a composition for improving skin condition comprising a plant peptone as an active ingredient.

It is still another object of this invention to provide a composition for improving skin condition comprising serum-free stem cell medium containing plant peptone removed of stem cells as an active ingredient.

Other objects and advantages of the present invention will become apparent from the detailed description to follow and together with the appended claims and drawings.

### Means for Solving Technical Problems

In one aspect of this invention, there is provided a serum-free medium composition for culturing a stem cell comprising a plant peptone.

In another aspect of this invention, there is provided a composition for improving skin condition comprising a plant peptone as an active ingredient

It is still another aspect of this invention, there is provided a composition for improving skin condition comprising serum-free stem cell medium containing plant peptone removed of stem cells as an active ingredient.

The present inventors have conducted intensive researches to find a material that can induce stem cell proliferation in a serum-free medium that can replace animal serum. As a result, the inventors have discovered that plant peptone was effective enough to be substitute with the animal serum for inducing stem cell proliferation, and thus completed the present invention.

This invention provides a serum-free medium composition free of animal serum comprising a plant peptone.

The above plant peptone has a great effect on inducing stem cell proliferation, thus can be used as an animal serum substitute in a serum-free stem cell culture medium.

The serum-free stem cell culture medium composition comprising plant peptone, wherein the plant peptone is contained in an appropriately selected amount possible to induce stem cell proliferation and not specifically limited to, but preferably in the range of 0.1-10% (g/ml) and more preferably 0.5-2% (g/ml).

According to the preferred embodiment, serum-free stem cell culture composition comprising plant peptone includes reduced amount of animal serum.

The composition comprises plant peptone as a serum substitute, therefore adding less than conventional amount of animal serum in stem cell culture medium is suitable for the stem cell culture. Preferably, the reduced amount of animal serum is less than 1/2 of the conventional amount of animal serum contained in the stem cell culture medium.

The term "plant peptone" used herein refers to a product by cleaving a plant-derived protein.

For example, soy peptone refers to a product by cleaving the total protein obtained from soy. Cleaving a plant-derived protein is preferably performed by partial digestion. The protein digestion is preferably through acid treatment, base treatment, enzymatic treatment, high-pressure treatment, heat treatment or physical treatment. The physical treatment is grinding.

The plant peptone in this invention refers to partial digestion product of plant-derived protein, which is in the form of a mixture that includes not only single molecule of amino acid, but also peptides consisting of few or several tens of amino acids and intact protein molecules.

The plant source for the plant peptone is not limited to, but may include any kind of plant-derived peptones having stem cell proliferation inducing ability.

Preferably, plant peptone in this invention is soy peptone, wheat peptone, broadbean peptone, potato peptone, pea peptone, Papaic soy peptone or lupin peptone, most preferably pea peptone and wheat peptone.

This invention provides a composition for improving skin condition comprising plant peptone as an active ingredient.

Since plant peptone exhibits excellent stem cell proliferation inducing ability, when applied on human skin, the plant peptone could improve skin condition by inducing adult stem cells in skin proliferation or activation. Adult stem cells in skin are, for example, stem cells in the basal layer of the interfollicular epidermis, stem cells in hair follicle and adipocyte-derived stem cell in subcutaneous layer.

According to the preferred embodiment, the plant peptone is contained in the amount of 0.01-30 wt% of the total composition. There is no effect on skin condition improvement when the amount of plant peptone is less than 0.01 wt% of the total composition. When the amount of plant peptone contained exceeds 30 wt%, it is difficult to achieve increased improvement compared to the increased amount of plant peptone. In addition, there is a down side of price increase for manufacturing.

Since the plant peptone contained in the present composition for improving skin conditions may be described with referring to descriptions of the plant peptone in the serum-free medium of the present invention, the common descriptions between them are omitted in order to avoid undue redundancy leading to the complexity of this specification.

According to the preferred embodiment, the improvement in skin condition includes amelioration of inflammatory skin diseases, improvement in skin wrinkle, inhibition of skin aging, improvement in skin elasticity, improvement in vitiligo, wound healing and skin regeneration.

According to another preferred embodiment, the inflammatory skin disease is atopic dermatitis, eczema, acne or psoriasis.

The composition for improving skin condition may be provided as functional food composition. In the functional food composition of this invention may include any conventional food ingredients, for example, protein, carbohydrate, fat, nutrition and seasoning. For example, in the formulation of drinks, it may comprise flavoring or natural carbohydrate in addition to an active ingredient, plant peptone. For example, the natural carbohydrate is monosaccharide (*e.g*., glucose, fructose etc.); disaccharide (*e.g*., maltose, sucrose etc.); oligosaccharide; polysaccharide (*e.g*., dextrin, cyclodextrin etc.); and sugar alcohol (*e.g*., xylitol, sorbitol, estritol etc.). For flavoring, natural flavoring (e.g., taurine, stevia extraction etc.) and artificial flavoring (*e.g*., saccharine, aspartame etc.) may be used. Considering the convenient accessibility to food, the functional food composition in this invention is valuable in improving skin condition.

According to the preferred embodiment, the functional food composition is a form selected from the group consisting of a solution, a suspension, an emulsion, a paste, a gel, a cream, a powder, a bar, a candy, an ice cream, an oil, a powdered emulsion and a spray.

The composition for improving skin condition may be provided in cosmetic composition applied directly on the skin.

The cosmetic composition of this invention may be formulated in a wide variety of form, for non-limited example, including a solution, a suspension, an emulsion, a paste, an ointment, a gel, a cream, a lotion, a powder, a soap, a surfactant-containing cleanser, an oil, a powder foundation, an emulsion foundation, a wax foundation and a spray. In detail, the cosmetic composition of the present invention can be provided in a form of skin softener (skin lotion), astringent lotion, nutrient emulsion (milk lotion), nutrient cream, message cream, essence, eye cream, cleansing cream, cleansing foam, cleansing water, facial pack, spray or powder.

The cosmetically acceptable carrier contained in the present cosmetic composition, may be varied depending on the type of the formulation. For example, the formulation of pastes, ointment, creams or gels may comprise animal and vegetable fats, waxes, paraffins, starch, tragacanth, cellulose derivatives, polyethylene glycols, silicones, benbonites, silica, talc, zinc oxide or mixtures of these ingredients.

In the formulation of powder or spray, the carrier may comprise lactose, talc, silica, aluminum hydroxide, calcium silicate, polyamide powder and mixtures of these ingredients. Spray may additionally comprise the customary propellants, for example, chlorofluorohydrocarbons, propane/butane or dimethyl ether.

In the formulation of solution and emulsion, the carrier may comprise solvent, solubilizer and emulsifier, for example water, ethanol, isopropanol, ethyl carbonate, ethyl acetate, benzyl alcohol, benzyl benzoate, propylene glycol, 1,3-butylglycol, oils, glycerol fatty esters, polyethylene glycol or fatty acid esters of sorbitan or mixtures of these substances.

In the formulation of suspension, the carrier may comprise liquid diluents, for example water, ethanol or propylene glycol, suspending agents, for example ethoxylated isosteary alcohols, polyoxyethylene sorbitol esters and poly oxyethylene sorbitan esters, microcrystalline cellulose, aluminum metahydroxide, bentonite, agar and tragacanth or mixtures of these substances.

The formulation of cleansing composition with surfactant may comprise aliphatic alcohol sulfate, aliphatic alcohol ether sulfate, sulfosucinnate monoester, isothinate, imidazolium derivatives, methyltaurate, sarcodnate, fatty acid amide ether sulfate, alkyl amido betain, aliphatic alcohol, fatty acid glyceride, fatty acid diethanolamide, vegetable oil, lanoline derivatives, ethoxylated glycerol fatty acid ester or mixtures of these ingredients.

The cosmetic composition of this invention, may contain auxiliaries as well as carrier. The non-limiting examples of auxiliaries include preservatives, antioxidants, stabilizers, solubilizers, vitamins, colorants, odor improvers or mixtures of these substances.

According to the preferred embodiment, the cosmetic composition is a form selected from the group consisting of a solution, a suspension, an emulsion, a paste, a gel, a cream, a lotion, a shampoo, a powder, a soap, a surfactant-containing cleanser, an oil, a powder foundation, an emulsion foundation, a wax foundation and a spray.

This invention provides a composition for improving skin condition comprising serum-free stem cell medium containing plant peptone removed of stem cells as an active ingredient.

The stem cells cultured in the present invention include, but not limited to, embryonic stem cells and adult stem cells, preferably mammalian adult stem cells, more preferably human adult stem cells, most preferably, human cord blood-derived mesenchymal stem cells (CB-MSCs), human skin-derived stem cells and human adipose-derived stem cells (ADSCs).

The term used herein "stem cell" means cells cell having potentials of self-cell cleavage and differentiation into various specific cell types.

The term used herein "Adult stem cell" refers to undifferentiated stem cells found ubiquitously in body after embryo development. The adult stem cells proliferated by cell cleavage may replace dead cells and regenerate damaged tissues. The adult stem cells generally possess site-specific differentiation potentials adaptable to surrounding tissues. The stem cell medium obtained by culturing adult stem cells has safety to human body and minimized adverse effects.

The removal of stem cells from media after stem cell culturing may be performed in accordance with conventional techniques. For instance, the stem cell media after stem cell culturing are retrieved and filtered through a micro syringe filter, thereby obtaining media removed of stem cells. The pore sizes of the micro syringe filter may be selected within sizes sufficient to remove stem cells, preferably 0.1-10 µm.

The amount of the plant peptone in the serum-free medium for stem cells may be selected within ranges sufficient to promote stem cell proliferation, preferably 0.1-10%(g/ml), more preferably 0.5-2%(g/ml).

The medium removed of stem cells contains no various biological contaminants derived from animal sera, *e.g*., animal bacteria, animal viruses, prion and toxins, such that the medium removed of stem cells may overcome problems of infection or toxicity associated with the contaminants.

The medium removed of stem cells after stem cell culture contains physiologically active substances secreted from stem cells (*e.g*., cytokines with abilities to promote proliferation and activation of stem cells). Therefore, where the medium removed of stem cells after stem cell culture is applied to human body, it contributes to improvement in skin conditions by promoting proliferation and activation of adult stem cells present in human body.

The adult stem cells in skin that are activated of induced to proliferate by the composition in this invention are stem cells in the basal layer of the interfollicular epidermis, stem cells in hair follicle and adipocyte-derived stem cell in subcutaneous layer.

According to the preferred embodiment, the stem cell culture medium is contained in the amount of 0.01-30 wt% of the total composition for improving skin condition. There is no effect on skin condition improvement when the amount of stem cell culture medium is less than 0.01 wt% of the total composition. When the amount of stem cell culture medium exceeds 30 wt%, it is difficult to achieve increased improvement compared to the increased amount of plant peptone. In addition, there is a down side of price increase for manufacturing.

Since the plant peptone contained in the present composition for improving skin conditions may be described with referring to descriptions of the plant peptone in the serum-free medium of the present invention, the common descriptions between them are omitted in order to avoid undue redundancy leading to the complexity of this specification.

According to the preferred embodiment, the improvement in skin condition includes amelioration of inflammatory skin diseases, improvement in skin wrinkle, inhibition of skin aging, improvement in skin elasticity, improvement in vitiligo, wound healing and skin regeneration.

According to another preferred embodiment, the inflammatory skin disease is atopic dermatitis, eczema, acne or psoriasis.

The composition for improving skin condition may be provided as functional food composition. In the functional food composition of this invention may include any conventional food ingredients, for example, protein, carbohydrate, fat, nutrition and seasoning. For example, in the formulation of drinks, it may comprise flavoring or natural carbohydrate in addition to an active ingredient, plant peptone. For example, the natural carbohydrate is monosaccharide (*e.g*., glucose, fructose etc.); disaccharide (*e.g*., maltose, sucrose etc.); oligosaccharide; polysaccharide (*e.g*., dextrin, cyclodextrin etc.); and sugar alcohol (*e.g*., xylitol, sorbitol, estritol etc.). For flavoring, natural flavoring (*e.g*., taurine, stevia extraction etc.) and artificial flavoring (*e.g*., saccharine, aspartame etc.) may be used. Considering the convenient accessibility to food, the functional food composition in this invention is valuable in improving skin condition.

According to the preferred embodiment, the functional food composition is a form selected from the group consisting of a solution, a suspension, an emulsion, a paste, a gel, a cream, a powder, a bar, a candy, an ice cream, an oil, a powdered emulsion and a spray.

The composition for improving skin condition may be provided in cosmetic composition applied directly on the skin.

The cosmetic composition of this invention may be formulated in a wide variety of form, for non-limited example, including a solution, a suspension, an emulsion, a paste, an ointment, a gel, a cream, a lotion, a powder, a soap, a surfactant-containing cleanser, an oil, a powder foundation, an emulsion foundation, a wax foundation and a spray. In detail, the cosmetic composition of the present invention can be provided in a form of skin softener (skin lotion), astringent lotion, nutrient emulsion (milk lotion), nutrient cream, message cream, essence, eye cream, cleansing cream, cleansing foam, cleansing water, facial pack, spray or powder.

The cosmetically acceptable carrier contained in the present cosmetic composition, may be varied depending on the type of the formulation. For example, the formulation of pastes, ointment, creams or gels may comprise animal and vegetable fats, waxes, paraffins, starch, tragacanth, cellulose derivatives, polyethylene glycols, silicones, bentonites, silica, talc, zinc oxide or mixtures of these ingredients.

In the formulation of powder or spray, the carrier may comprise lactose, talc, silica, aluminum hydroxide, calcium silicate, polyamide powder and mixtures of these ingredients. Spray may additionally comprise the customary propellants, for example, chlorofluorohydrocarbons, propane/butane or dimethyl ether.

In the formulation of solution and emulsion, the carrier may comprise solvent, solubilizer and emulsifier, for example water, ethanol, isopropanol, ethyl carbonate, ethyl acetate, benzyl alcohol, benzyl benzoate, propylene glycol, 1,3-butylglycol, oils, glycerol fatty esters, polyethylene glycol or fatty acid esters of sorbitan or mixtures of these substances.

In the formulation of suspension, the carrier may comprise liquid diluents, for example water, ethanol or propylene glycol, suspending agents, for example ethoxylated isosteary alcohols, polyoxyethylene sorbitol esters and poly oxyethylene sorbitan esters, micocrystalline cellulose, aluminum metahydroxide, bentonite, agar and tragacanth or mixtures of these substances.

The formulation of cleansing composition with surfactant may comprise aliphatic alcohol sulfate, aliphatic alcohol ether sulfate, sulfosucinnate monoester, isothinate, imidazolium derivatives, methyltaurate, sarcocinate, fatty acid amide ether sulfate, alkyl amido betain, aliphatic alcohol, fatty acid glyceride, fatty add diethanolamide, vegetable oil, lanoline derivatives, ethoxylated glycerol fatty acid ester or mixtures of these ingredients.

The cosmetic composition of this invention, may contain auxiliaries as well as carrier. The non-limiting examples of auxiliaries include preservatives, antioxidants, stabilizers, solubilizers, vitamins, colorants, odor improvers or mixtures of these substances.

According to the preferred embodiment, the cosmetic composition is a form selected from the group consisting of a solution, a suspension, an emulsion, a paste, a gel, a cream, a lotion, a shampoo, a powder, a soap, a surfactant-containing cleanser, an oil, a powder foundation, an emulsion foundation, a wax foundation and a spray.

### Advantages of This Invention

Since the serum-free medium for culturing stem cells containing the plant peptone uses no high-cost animal serum, it shows very cost-effectiveness and prevents contamination of animal materials associated serum, thereby providing more safe stem cells. In addition, not only the composition containing the plant peptone but also the medium after stem cell culture in the serum-free medium containing the plant peptone has potentials of promoting stem cell proliferation and activation, exhibiting various efficacies such as amelioration of inflammatory skin diseases, improvement in skin wrinkle, inhibition of skin aging, improvement in skin elasticity, improvement in vitiligo, wound healing or skin regeneration.

### BRIEF DESCRIPTION OF THE DRAWINGS

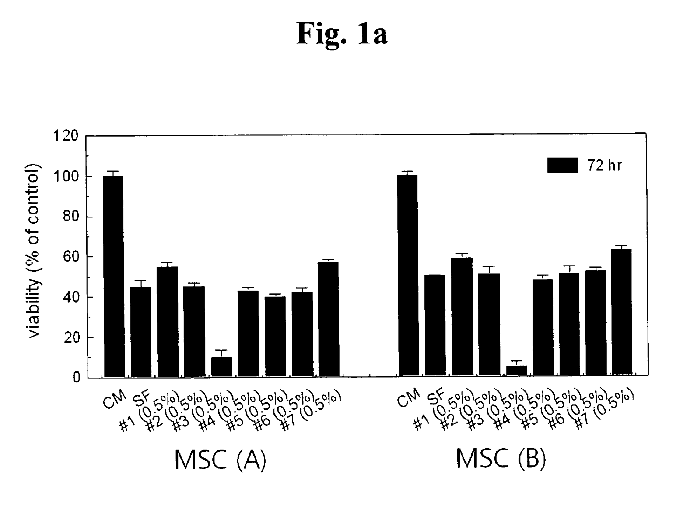

Fig. 1a is a graph showing the viability assay result of human cord blood-derived mesenchymal stem cells (CB-MSCs) grown in serum-free media with 7 different kinds of plant peptones. CM : DMEM containing 15% FBS and 1% antibiotics; SF: serum-free DMEM; MSC: human cord blood-derived mesenchymal stem cells with different cell passage numbers; #1:pea peptone, #2:Lupin peptone, #3:potato peptone, #4:soy peptone, #5: papaic soy peptone, #6: broadbean peptone, #7:wheat peptone. The concentration of peptone is 5mg/ml (0.5%)

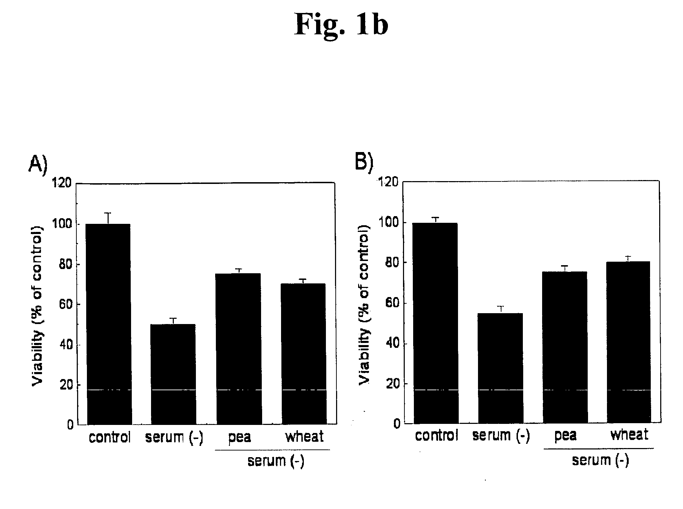

Fig. 1b are graphs showing the effect of peptone on the viability of human cord blood-derived mesenchymal stem cells (CB-MSCs) or human adipose-derived stem cells (ADSCs). Adult stem cells were grown in recombinant culture medium containing pea peptone or wheat peptone for 3 days. For serum-free condition, the cell medium was replaced with serum-free DMEM. For positive control groups, cells were incubated in control medium (DMEM medium containing 15% FBS). Cell viability was assessed by MTT analysis. The experimental values are indicated as a percentage rate of control. The experimental value for each group is the mean value of 4 samples (n=4). Panel A is the viability result from CB-MSCs and panel B is the viability result from ADSCs.

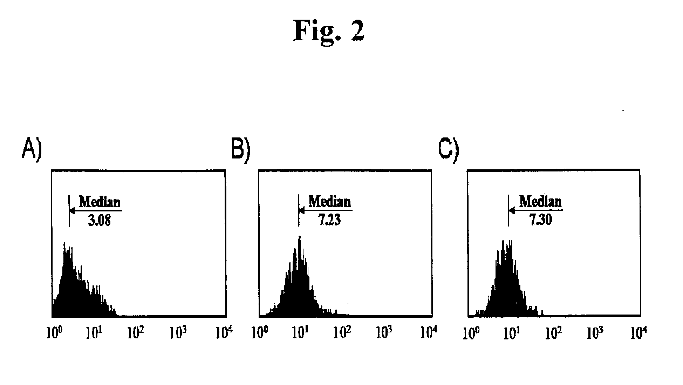

Fig. 2 is graphs showing the effect of peptone on cell proliferation. CB-MSCs were grown in recombinant culture medium containing pea peptone or wheat peptone for 3 days. Cell proliferation was evaluated with Click-iT^{™} EdU flow cytometry assay. Samples were analyzed by FACS Vantage flow cytometer. Panel A is the serum-free condition; panel B is serum-free condition containing pea peptone; panel C is serum-free condition containing wheat pepton.

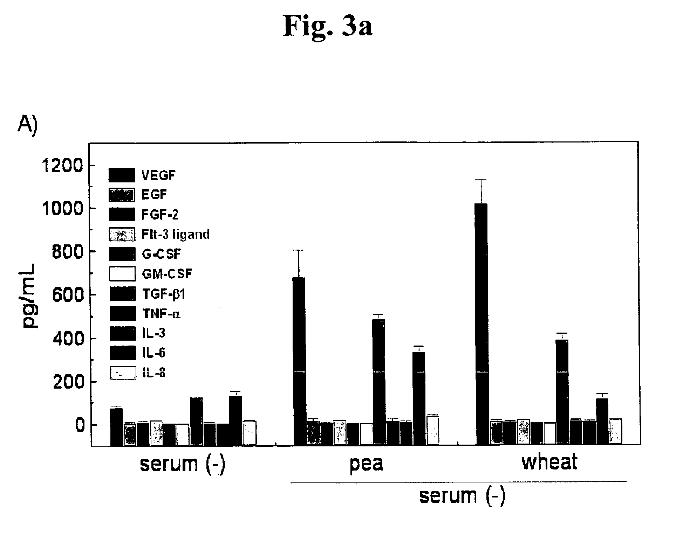

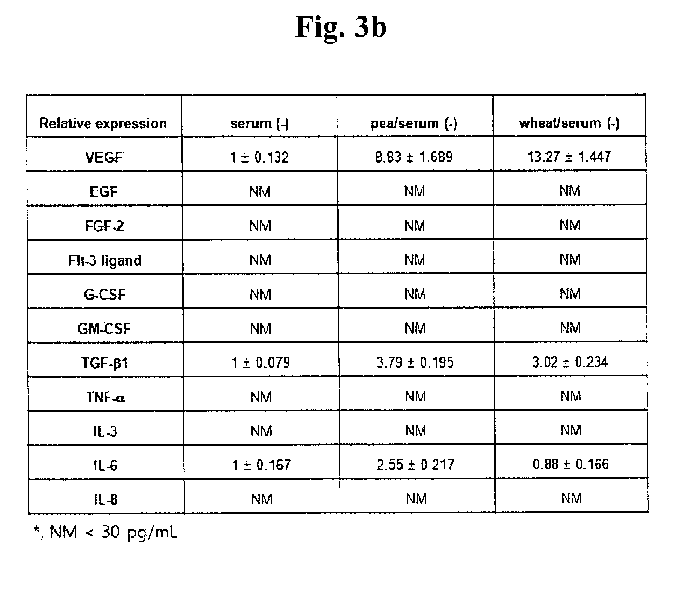

Figs. 3a and 3b are results representing the cytokine profiles of human cord blood-derived mesenchymal stem cells (CB-MSCs) in controlled medium treated with plant peptone. CB-MSCs were grown in culture medium containing pea peptone or wheat peptone for 3 days. Cytokines were quantified by culturing the human cord blood-derived mesenchymal stem cells (CB-MSCs) in serum-free condition with or without peptones and analyzing the supernatant of the culture medium. The experimental value in each group is the mean value of 4 samples (n=4). * NM means cytokine production of < 30 pg/ml after peptone treatment.

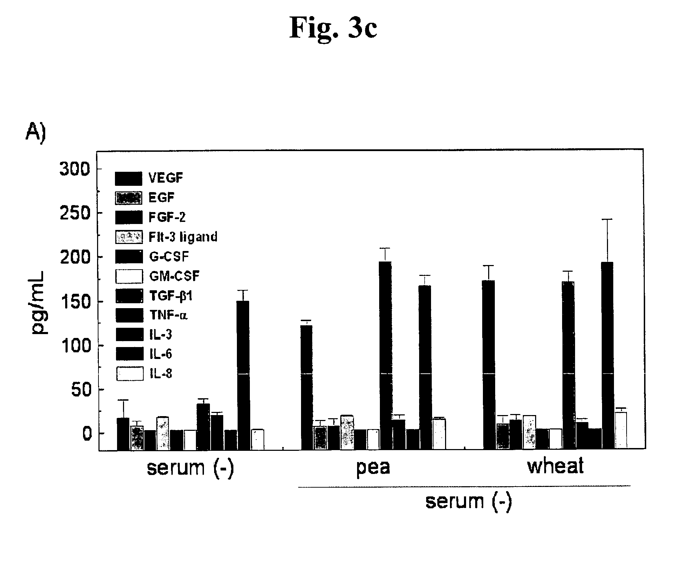

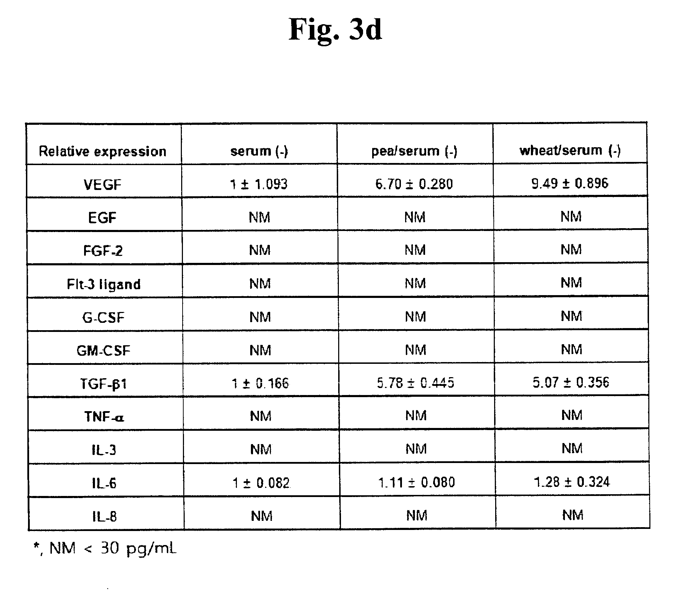

Figs. 3c and 3d are results representing the cytokine profiles of human adipose-derived stem cells (ADSCs) in controlled medium treated with plant peptone. Human adipose-derived stem cells (ADSCs) were grown in culture medium containing pea peptone or wheat peptone for 3 days. Cytokines were quantified by culturing the human adipose-derived stem cells (ADSCs) in serum-free condition with or without peptones and analyzing the supernatant of the culture medium. The experimental value in each group is the mean value of 4 samples (n=4). * NM means cytokine production of < 30 pg/ml after peptone treatment.

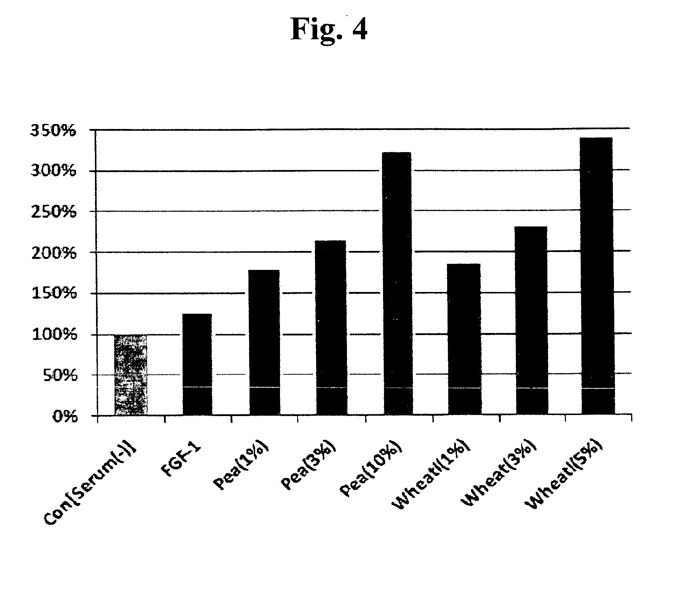

Fig. 4 is a graph representing the effect of plant peptone on inducing fibroblast proliferation. Fibroblasts were grown in culture medium containing pea peptone or wheat peptone for 3 days. After 72 hrs of incubation, the cells were washed once with serum-free medium and incubated for 3 hrs in serum-free medium containing 10% MTT before measuring the O.D. value and converting into percentage rate. Con: serum-free condition, FGF-1: Fetal Growth Factor-1, Pea: pea peptone, Wheat: wheat peptone.

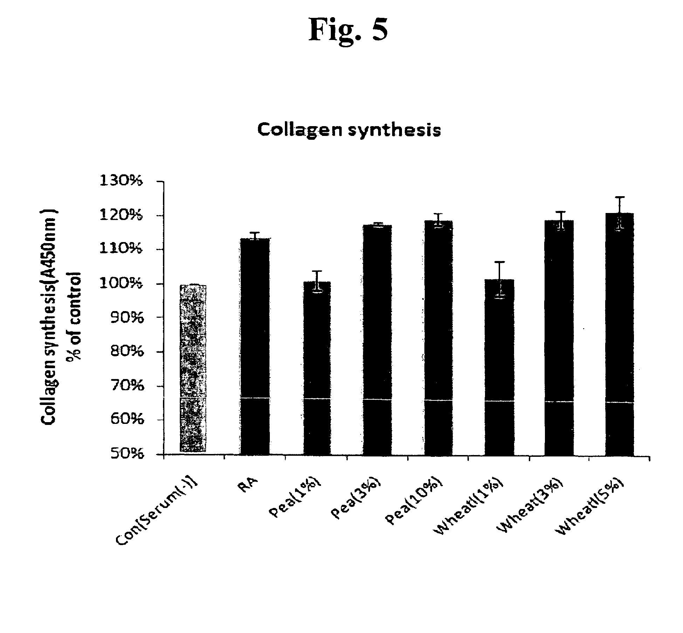

Fig. 5 is a graph representing the effect of plant peptone on inducing collagen synthesis in fibroblasts. Human normal fibroblasts (Taepyungyang, Korea) were inoculated in a 6-well plate (2 x 10⁵ cells/well) containing DMEM and incubated for 24 hrs at 37°C under 5% CO₂. After 24 hrs, the medium was removed and the cells were incubated for another 24 hrs in the medium with Retinoic acid (RA) in positive control group, medium with 1, 3, and 10% of pea peptone or 1, 3, 5% of wheat peptone in experimental groups and serum-free medium in negative control group. The samples were prepared by collecting cell medium after 24 hrs. The level of collagen synthesis was determined by measuring the amount of Procollagen Type I C-peptide (PICP) using Procollagen Type I C-peptide EIA kit (MK101; Takara, Kyoto, Japan). The Takara kit was used according to manufacturer's protocol. The median value is shown as mean ± SD. The data was analyzed by SPSS/PC+ program and t-test was done to test for significance. Con: serum-free condition, RA: Retinoic acid, Pea: pea peptone, Wheat: wheat peptone.

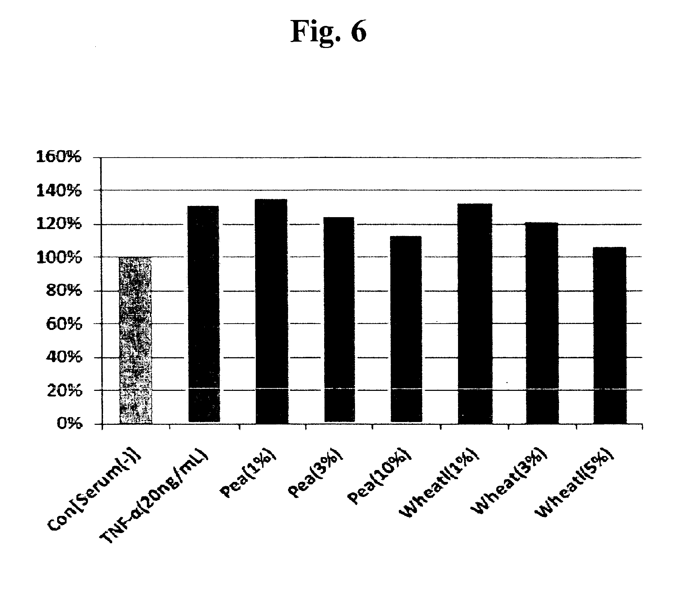

Fig. 6 is a graph showing the inhibitory effect of plant peptone on collagen degrading enzyme. Human normal fibroblasts (Taepyungyang, Korea) were inoculated in 6-well plate (2 x 10⁵ cells/well) containing DMEM and incubated for 24 hrs at 37°C under 5% CO₂. After 24 hrs, the medium was removed, treated with concentration gradients of samples then incubated for another 24 hrs. Samples were prepared by collecting cell medium. The activity of collagenase, the collagen degrading enzyme, was measured using an antibody against collagen. PMA (phrobol myristate acetate) was used to induce the collagenase activity. A commercially available kit was used according to manufacturer's protocol. Type I collagenase kit (Amersham Biosciences, RPN2629) was used and the absorbance was read with an ELISA reader. The median value is shown as mean ± SD. The data was analyzed by SPSS/PC+ program and t-test was done to test for significance. Con: serum-free condition, TNF-α: Tumor necrosis Factor- α, Pea: pea peptone, Wheat: wheat peptone.

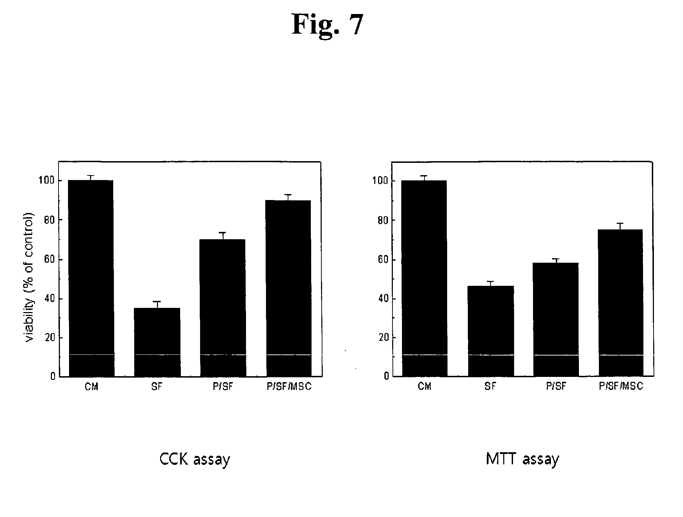

Fig. 7 is graphs showing the activation of human epidermal stem cells in epidermis by plant peptone. CM: DMEM with 15% FBS and 1%; SF: serum-free DMEM, P/SF: serum-free DMEM with plant peptone (pea peptone); P/SF/MSC, culture medium of human cord blood-derived mesenchymal stem cells cultured in serum-free medium with plant peptone (pea peptone).

### EXAMPLES

The present invention will now be described in further detail by examples. It would be obvious to those skilled in the art that these examples are intended to be more concretely illustrative and the scope of the present invention as set forth in the appended claims is not limited to or by the examples.

### Example 1: Cell viability assay of stem cell cultured in a serum-free medium containing plant peptone

### Cell, medium and culture method

In order to find a serum substitute in the stem cell culture media, the inventors screened for a reagent promoting stem cell proliferation by adding plant composition in the serum-free media. Based on the origin, amino add content and molecular weight distribution of the peptone, the following seven peptones were selected for the experiment; soy peptone, wheat peptone, broadbean peptone, potato peptone, pea peptone, papaic soy peptone and lupin peptone.

For the stem cells, human cord blood-derived mesenchymal stem cells (CB-MSCs) and human adipocyte-derived stem cells were used.

Human cord blood-derived mesenchymal stem cells (CB-MSCs) were provided by College of Medicine, Pochon CHA University (Seongnam, Korea). Cells were plated with DMEM (Dulbecco's modified eagle medium) containing a low level of glucose, 5% FBS, penicillin and streptomycine and then incubated at 37°C. The medium was replaced every 3 days until the cells reached confluence. Human adipose-derived stem cells (ADSCs) were purchased from Invitrogen (Invitrogen, Carlsbad, CA, USA). Frozen cells were thawed at 37°C and then plated in MesenPRO RS™ medium (Gibco, Carlsbad, CA, USA). Cells were further proliferated further in MesenPRO RS™ medium. Soy peptone, wheat peptone, broadbean peptone, potato peptone and pea peptone were purchased from Flucka (Sigma-Aldrich, St Quentin Fallavier, France). Papaic soy peptone and lupin peptone were purchased from Solabia (Pantin 93698 Cedex, France). Peptones were filtered through 0.2 µm membrane filter before being added to the serum-free DMEM medium at 1% peptone concentration. In the serum-free negative control groups, the cell medium was replaced with serum-free DMEM (Cat. 11885, Invitrogen). For positive control groups, cells were incubated in DMEM medium only containing 15% FBS.

### Cell viability assay

Cell viability was assessed by MTT (3-(4,5-dimethylthiazol-2-yl)-2,5-diphenyltetrazolium bromide) analysis. MTT assay measures the mitochondrial dehydrogenase activity as it reduces tetrazolium rings to blue colored formazan. The level of blue formazan can be quantified by measuring with spectrophotometer and can be used as an indirect means of determining the cell density. Briefly, cells were exposed to MTT (1 mg/ml) for 3 hrs, then the medium was removed and the cells were dissolved in DMSO (dimethyl sulfoxide). After completely dissolving, the blue formazan was quantified by the absorbance at 570 nm by a spectrophotometer.

When the effect of peptone on adult stem cell proliferation was analyzed using MTT assay, 2 of the plant peptones, pea peptone and wheat peptone induced high increase in adult stem cell survival (Fig. 1a). Pea peptone and wheat peptone greatly increased the growth rate of cord blood-derived mesenchymal stem cells (CB-MSCs) by 25% and 20%, respectively (Fig 1b, panel A), similarly, pea peptone and wheat peptone increased the cell growth rate of adipose-derived stem cells (ADSCs) by 20% and 25% respectively (Fig. 1b, panel B).

### Cell proliferation evaluation

The effect of pea and wheat peptones on the viability of the stem cells was additionally confirmed by evaluating the cell proliferation of cord blood-derived mesenchymal stem cells (CB-MSCs).

Cell proliferation evaluation is based on detecting ³H thymidine or bromodeoxyuridine (BrdU) derivatives incorporated during the DNA synthesis. Click-iT^{™} EdU flow cytometry assay kit is a new procedure replacing assays using BrdU. Click-iT^{™} EdU assay uses a modified nucleoside of thymidine, EdU (5-ethynyl-2'-deoxyuridine), instead of traditional method of using nucleoside derivative, BrdU incorporated during the DNA synthesis to be used as a base for antibody detection.

Briefly, the cells were incubated with EdU for 1 hr. Negative controls were cells from the same group without EdU treatment Cells were analyzed by FACS Vantage flow cytometer (BD Biosciences, San Jose, CA, USA). All the analysis procedures were performed according to manufacturer's protocol.

Cell proliferation analysis of cord blood-derived mesenchymal stem cells (CB-MSCs), as shown in Fig. 2 represents the median values of groups treated with pea peptone or wheat peptone. Experimental groups show a shift to the right compared to the serum-free sample group (median value of 3.08 shifted to 7.23 and 7.30, respectively).

### Implication of the experimental result

Based on above results, the plant peptones are suggested to contribute greatly to proliferation of stem cells grown in serum-free medium.

### Example 2: Composition and characteristics of plant peptone

The physiochemical characteristics of pea peptone and wheat peptone were investigated. Physical characteristics were investigated according to conventional methods. Microbiological tests were performed according to a conventional method. Amino acid composition were analyzed by conventional amino acid analyzer.

### Pea peptone

a. Physical properties : The appearance is a light brown powder, which is stable in a 2% solution, solubility approaches 5% of total solution.
b. Micobiological controls are aerobic/mesophilic and flora (total mesophilic flora) ≤≤ 5000 cfu/g.
c. Amino acid distribution (mg/g) is shown as Table 1.

**Table 1**

| Pea peptone | Total amino acids (T) | Free amino acids (F) | (F/T)x 100 | | Total amino acids(T) | Free amino acids (F) | (F/T)x 100 |
|---|---|---|---|---|---|---|---|
| Aspartic acid | 103.37 | 2.31 | 2.23 | Methionine | 0.00 | 2.25 | 0.00 |
| Threonine | 32.28 | 7.56 | 23.42 | Isoleucine | 38.68 | 8.25 | 21.33 |
| Serine | 48.71 | 6.12 | 12.56 | Leucine | 60.10 | 19.49 | 32.43 |
| Glutamic acid | 169.25 | 4.84 | 2.86 | Tyrosine | 20.93 | 9.84 | 47.01 |
| Proline | 38.83 | 0.93 | 2.40 | Phenylalanine | 42.01 | 14.54 | 34.61 |
| Glycine | 37.93 | 4.19 | 11.05 | Histidine | 19.64 | 4.11 | 20.93 |
| Alanine | 37.08 | 4.94 | 13.32 | Lysine | 68.50 | 14.46 | 21.11 |
| cysteinin | 49.77 | 3.22 | 6.47 | Arginine | 80.56 | 25.72 | 31.93 |
| Valine | 40.96 | 9.30 | 22.72 | Tryptophan | 12.69 | 3.39 | 26.71 |

### Wheat peptone

a. Physical properties: The appearance is a light brown powder, which is stable in a 2% solution, solubility approaches 5% of total solution.
b. Micobiological controls are aerobic/mesohilic and flora (total mesophilic flora) is ≤≤ 5000 cfu/g.
c. Amino acid distribution (mg/g) is shown as Table 2.

**Table 2**

| Pea peptone | Total amino acids(T) | Free amino acids(F) | (F/T)x 100 | | Total amino acids (T) | Free amino adds (F) | (F/T)x 100 |
|---|---|---|---|---|---|---|---|
| Aspartic acid | 17.52 | 2.11 | 12.04 | Methionine | 5.83 | 2.64 | 45.28 |
| Threonine | 15.27 | 2.88 | 18.86 | Isoleucine | 24.45 | 3.17 | 12.97 |
| Serine | 31.51 | 5.12 | 16.25 | Leucine | 44.07 | 10.38 | 23.55 |
| Glutamic add | 245.63 | 3.46 | 1.41 | Tyrosine | 11.72 | 4.04 | 34.47 |
| Proline | 82.54 | 0.35 | 0.42 | Phenylalanine | 33.66 | 3.56 | 10.58 |
| Glycine | 19.50 | 1.95 | 10.00 | Histidine | 11.19 | 1.63 | 14.57 |
| Alanine | 15.50 | 2.46 | 15.87 | Lysine | 12.60 | 2.74 | 21.74 |
| Cysteinin | 32.69 | 1.67 | 5.11 | Arginine | 18.30 | 5.11 | 27.92 |
| Valine | 22.84 | 4.76 | 20.84 | Tryptophan | 4.49 | 5.57 | 124.05 |

### Example 3: cell viability assay of animal cells grown in serum-free medium containing plant peptone

To understand whether plant peptone can induce cell proliferation in other animal cells, human keratinocyte, HaCaT and mouse fibroblast, NIH3T3 cells were grown in serum-free media containing plant peptone to assess the effect of plant peptone on cell viability. The experiment was performed as follows. HaCaT and NIH3T3 cells grown in DMEM containing 10% FBS and 1% antibiotics at 37°C under 5% CO₂ were inoculated and plated in 6-well plates at 3 x 10⁵ cells per well. The following day, cells were washed once with serum-free medium then incubated in different experimental media groups, either in serum-free medium or in serum-free medium with plant peptone. After 72 hrs of incubation, the cells were washed once with serum-free medium and incubated for 3 hrs in serum-free medium containing 10% MTT before measuring the O.D. value and converting into percentage rate.

As shown in Fig. 3 below, compared to a cell viability of 100% in the control group (serum containing medium), pea peptone and wheat peptone containing group both showed more than 70% of increase in cell viability compared to the serum-free group (54%). The result demonstrate that wheat peptone and pea peptone can induce cell proliferation in HaCaT and 3T3 cells as well as in stem cells.

**Table 3**

| | | Serum-free medium | Serum medium | Serum-free medium containing pea peptone | Serum-free medium containing wheat peptone |
|---|---|---|---|---|---|
| Cell | HaCaT | 54% | 100% | 72% | 75% |
| Viability | NIH3T3 | 52% | 100% | 75% | 76% |

### Example 4: Investigating the different expression profiles of growth stimulating factors from stem cells cultured in serum-free medium containing plant peptone

In Example 1, experiments confirmed that pea peptone and wheat peptone induce adult stem cell proliferation. In addition, the mechanism of plant peptone on stem cell proliferation was confirmed.

The expression profile of cytokines, EFG, FGF-2, Flt-3 ligand, G-CSF, GM-CSG, TGF-β1, IL-3, IL-6, IL-8, TNF-α and VEGF in response to plant peptones from adult stem cell was investigated by adding pea peptone and wheat peptone in the medium.

Adult stem cells (human cord blood-derived mesenchymal stem cells or human adipocyte-derived stem cells) cells grown in DMEM containing 10% FBS and 1% antibiotics at 37°C under 5% CO₂ were inoculated and plated on 6-well plates at 3 x 10⁵ cells per well. Cells were washed once with serum-free medium then incubated in different experimental groups, either in serum-free medium or in serum-free medium with plant peptone (pea peptone or wheat peptone). To measure cytokine quantities, the supernatant of stem cell cultured medium was collected after 72 hrs and were determined by the MILLIPLEX^{™} Human Cytokine Kit (Millipore, St. Charles, MO, USA) according to the manufacturer's protocol.

As presented in Figs 3a-3d, there was significant increases in production of cytokines, VEGF, TGF-β1 and IL-6 when adult stem cells were incubated with plant peptone.

According to the detailed result in Fig. 3a and Fig. 3b, the amount of VEGF produced was 678 pg/ml from human cord blood-derived mesenchymal stem cells (CB-MSC) treated with pea peptone, which was 8.83 ± 1.689 times higher compared to untreated control; the production of TGF-β1 was 485 pg/ml, which was 3.79 ± 0.195 times higher than the untreated control; the production of IL-6 was 332 pg/ml, which was 2.55 ± 0.217 times higher than the untreated control. When treated with wheat peptone, VEGF amount was 1019 pg/ml, which was 13.27 ± 1.447 times higher compared to untreated control; the production of TGF-β1 was 387 pg/ml, which was 3.02 ± 0.234 times higher than the untreated control; however the production of IL-6 did not change in human cord blood-derived mesenchymal stem cells treated with wheat peptone.

According to the detailed result in Fig. 3c and Fig. 3d, the amount of VEGF produced was 122 pg/ml from human adipocyte-derived stem cells treated with pea peptone, which was 6.70 ± 0.280 times higher compared to untreated control; the production of TGF-β1 was 194 pg/ml, which was 5.78 ± 0.445 times higher than the untreated control; the production of IL-6 was 166 pg/ml, which was 1.11 ± 0.080 times higher than the untreated control. When human adipocyte-derived stem cells were treated with wheat peptone, the amount of VEGF produced was 172 pg/ml, which was 9.49 ± 0.896 times higher compared to the untreated control; the production of TGF-β1 was 170 pg/ml, which was 5.07 ± 0.356 times higher than the untreated control; the production of IL-6 was 192 pg/ml which was 1.28 ± 0.324 times of increase compared to the untreated group.

Except for VEGF, TGF-β and IL-6, there was no detectable change in the secretion level of other cytokines from human cord blood-derived mesenchymal stem cells (CB-MSCs) or human adipocyte-derived stem cells treated with pea peptone and wheat peptone. Therefore, the induction of stem cell proliferation by plant peptone is considered to be mediated by VEGF, TGF-β and IL-6.

### Example 5: Cell viability assay of human normal fibroblasts cultured in serum-free medium containing plant peptone

The effect of plant peptone on cell viability was assessed in normal human fibroblasts. Normal human fibroblasts grown in DMEM containing 10% FBS and 1% antibiotics at 37°C under 5% CO₂ were inoculated and plated in 6-well plate at 3 x 10⁵ cells per well. The following day, cells were washed once with serum-free medium and incubated in different experimental groups, either in serum-free medium or in serum-free medium with peptone (1-10%). After 72 hrs of incubation, the cells were washed once with serum-free medium and incubated for 3 hrs in serum-free medium containing 10% MTT before measuring the O.D. value and converting into percentage rate. As shown in the experimental result of Fig. 4, wheat peptone and pea peptone containing groups showed higher induction of cell proliferation in all treatment group concentrations as compared to FGF-1 (Fetal Growth Factor-1) treated positive control group. Therefore, the result showed that plant peptone can induce fibroblast cell proliferation.

### Example 6: Effect of plant peptone on induction of collagen synthesis in fibroblast

Induction of collagen biosynthesis in fibroblasts is an important indicator of prevention and reduction of wrinkles in the skin. Wrinkles are generally known to appear from the reduction of collagen biosynthesis and degradation of existing collagens. Therefore, induction of collagen synthesis in epidermal layer can lead to inhibition of wrinkles as well as improvement on wrinkles that have already formed.

Human normal fibroblasts (Taepyungyang, Korea) were inoculated in DMEM containing 6-well plate (2 x 10⁵cells/well) and incubated for 24 hrs at 37°C under 5% CO₂. After 24 hrs, the medium was removed and the cells were incubated for another 24 hrs in the medium with Retinoic acid (RA) in positive control group, medium with 1, 3, and 10% of pea peptone or 1, 3, 5% of wheat peptone in experimental groups and serum-free medium in negative control group. The samples were prepared by collecting cell medium after 24 hrs. The level of collagen synthesis was determined by measuring the amount of Procollagen Type I C-peptide (PICP) using Procollagen Type I C-peptide EIA kit (MK101; Takara, Kyoto, Japan). The experiment was performed according to manufacturer's protocol of the kit The median value is shown as mean ± SD. The data was analyzed by SPSS/PC+ program and t-test was done to test for significance. This result is shown in Fig. 5.

According to the result in Fig. 5, plant peptones (wheat peptone and pea peptone) increased the collagen synthesis in fibroblasts to the level similar to the positive control with Retinoic acid (RA).

### Example 7: Inhibitory effect of plant peptone on collagenase activity

Collagen is a structural protein of epidermal layer responsible for wrinkles when its level decreases. In opposite, wrinkle formation can be prevented when the level of collagen made are well maintained. Therefore, inhibiting collagenase activity is an important physiological indictor in anti-aging.

Human normal fibroblasts (Taepyungyang, Korea) were inoculated in DMEM containing 6-well plate (2 × 10⁵ cells/well) and incubated for 24 hrs at 37°C under 5% CO₂. After 24 hrs, the medium was removed, treated with concentration gradients of samples then incubated for another 24 hrs. Samples were prepared by collecting cell medium. The activity of collagenase, the collagen degrading enzyme, was measured using an antibody against collagen. PMA (phrobol myristate acetate) was treated as a reagent to induce the collagenase activity. A commercially available kit was used according to manufacturer's protocol. Type I collagenase kit (Amersham Biosciences, RPN2629) was used and the absorbance was read with an ELISA reader. The median value is shown as mean ± SD. The data was analyzed by SPSS/PC+ program and t-test was done to test for significance. This result is shown in Fig. 6. TNF (tumor necrosis factor)-α treated group was used as a positive control. Wheat peptone and pea peptone showed inhibitory effects on collagenase activity at treatment concentrations of 1% and higher.

### Example 8: Cell viability assay of epidermal stem cell using stem cell culture medium containing plant peptone

The culture medium of stem cells cultured in serum-free medium containing plant peptones were treated to epidermal stem cells to assess the activation level of the cells.

The culture medium of stem cells cultured in serum-free medium containing plant peptone in this Example is prepared as follows. Human cord blood-derived mesenchymal stem cells cultured in DMEM containing 15% FBS and 1% antibiotics at 37°C under 5% CO₂ were inoculated and plated at 3 x 10⁵ cells in 6-well plate. Cells were washed with serum-free medium then incubated in serum-free DMEM medium containing 1% plant peptone (pea peptone or wheat peptone) for 20 days. The culture medium was collected and filtered through 0.2 µm micro syringe filter to remove cells before using the medium.

The epidermal stem cell used in this Example was isolated according to the isolation method disclosed in Korean Journal of Investigative Dermatology (2006: 13 (1):1-4). Epidermal stem cell was isolated from cells collected from epidermal structure or epidermis that adheres to the type 4 collagen. The epidermal stem cell isolated were inoculated and plated at 3 x 10⁵ cells in 6-well plate, washed once with serum-free medium, then incubated into different experimental groups with various medium conditions as follows; normal control group with conventional level of serum (CM); serum-free group (SF), serum-free with 1% plant peptone (pea peptone or wheat peptone) group (P/SF); culture medium from human cord blood-derived mesenchymal stem cells grown in serum-free medium with 1% plant peptone (pea peptone or wheat peptone) treated group after its stem cells removed (P/SF/MSC). After incubating of 72hrs, MTT assay was performed and cell culture medium was collected for CKK (cell viability assay method) analysis.

As presented in Fig. 7, considering the normal control group as 100% (CM), there was a significant increase in cell viability in epidermal stem cells treated with serum-free medium with plant peptone (P/SF) and with culture medium from stem cells grown in serum-free medium with plant peptone (P/SF/MSC), when compared to serum-free treated group (SF).

### Example 9: Preparation of topical composition comprising plant peptone for improving skin condition

The test product was prepared as Oil in Water type emulsion which is the general form when plant peptones are used for topical skin composition, such as cosmetics or ointments. The component of each test products is shown in Table 4. The water phase, which includes distilled water, triethanolamine and propylene glycol were dissolved by heating at 70°C. This solution was added and emulsified with the lipid phase fatty acid, oily compound, emulsifier and preservative that were dissolved by heating at 70°C. After emulsifying, the solution was cooled to 45°C before adding 0.1% of plant peptone.

**Table 4**

| component | Amount (wt%) |
|---|---|
| Stem cell culture medium cultured in serum-free medium without peptone (control group 1) | 0.1 |
| Stem cell culture medium cultured in serum-free medium with pea peptone (experimental group 1) | |
| stem cell culture medium cultured in serum-free medium with wheat peptone (experimental group 2) | |
| Control group without peptone (control group 2) | |
| pea peptone (experimental group 3) | |
| Wheat peptone (experimental group 4) | |
| Jojoba oil | 5.0 |
| Liquid paraffin | 7.0 |
| Cetyl aryl alcohol | 2.0 |
| Polyglyceryl-3 methylglucose distearate | 2.0 |
| Glyceryl stearate | 0.5 |
| squalene | 3.0 |
| Propylene glycol | 4.0 |
| glycerin | 5.0 |
| Triethanolamine | 0.3 |
| Carboxyvinylpolymer | 0.3 |
| Tocopheryl acetate | 0.2 |
| Preservative, fragrance | Trace amount |
| Distilled water | Residual amount |
| **Total** | 100 |

### Example 10: Preparation of topical composition comprising culture medium of stem cells cultured with plant peptone for improving skin condition

Table 4 represents the composition of a cream containing the culture medium depleted of stem cells after growing stem cells (human cord blood-derived mesenchymal stem cells, CB-MSCs) in serum-free medium added with 1% plant peptone (pea peptone or wheat peptone). The cream composition was prepared as the method described in Example 9. At the last step, the culture medium from the stem cells grown in serum-free medium treated with plant peptone were added with fragrance, aliquoted and cooled to 30°C.

### Example 11: The effect of the composition comprising plant peptone or culture medium of stem cell cultured with plant peptone on improving acne

The effect of the stem cell (human cord blood-derived mesenchymal stem cells, CB-MSCs) culture medium cultured in medium containing 1% plant peptone depleted of stem cells and plant peptone (pea peptone or wheat peptone) alone on improving inflammatory skin condition were clinically tested. The cream containing 0.1% each of stem cell culture medium, wheat peptone or pea peptone was applied on the left side of the test subject's face. Cream depleted of culture medium or cream without peptone/wheat peptone was applied on the other side of the face as control. The improvement of acne was assessed by the number of comedos for 1 month. There was an improvement of the symptoms only on the left side of the face where cream containing stem cell culture medium and cream containing wheat peptone and pea peptone were applied.

The evaluation of acne was performed in conditioned environment at room temperature of 24-26°C and constant humidity of 38-40%. Patients with acnes (40 patients) were applied with 4 different type of creams (experimental group 1, 2, 3, 4) and control cream formulation (control group 1 and 2) as shown in Table 4, on either side of the face for twice/day for one month to assess the improvement of acne. The assay was performed by counting the number of acnes by drawing an imaginary grid with an area of three square centimeters, two centimeters below the eye. The result is shown in Table 5 below.

**Table 5**

| Test group | Cream with serum-free stem cell culture medium without peptone | Cream with serum-free stem cell culture medium with pea peptone | Cream with serum-free stem cell culture medium with wheat peptone |
|---|---|---|---|
| | (control group 1) | (experimental group 1) | (experimental group 2) |
| Number of comedos | 25.0 ± 2.1 | 7.5 ± 1.2 | 5.8 ± 2.1 |

| Test group | Cream without peptone | Cream with pea peptone | Cream with wheat peptone |
|---|---|---|---|
| | (control group 2) | (experimental group 3) | (experimental group 4) |
| Number of comedos | 32 ± 3.4 | 4.8 ± 1.3 | 4.2 ± 2.0 |

As shown in Table 5, the cream in this invention showed better improvement in acne compared to control cream 1 and 2.

### Example 12: The effect of the composition comprising plant peptone or culture medium of stem cells cultured with plant peptone on improving skin inflammation

The effect of the stem cell (human cord blood-derived mesenchymal stem cells, CB-MSCs) culture medium cultured in medium containing 1% plant peptone depleted of stem cells and plant peptone (pea peptone or wheat peptone) alone on improving chronic inflammatory skin condition, such as atopic dermatitis and psoriasis were clinically tested. The cream containing 0.1% each of stem cell culture medium, wheat peptone or pea peptone was applied on the left side of the face for each test subject. Cream depleted of culture medium or cream without peptone/wheat peptone was applied on the other side of the face as control. The improvement of typical inflammatory skin disease, atopic dermatitis was assessed by grading the level of erythema diminished. The result indicated an improvement in groups applied with cream containing stem cell culture medium and cream containing wheat peptone and pea peptone.

The evaluation of atopic dermatitis was performed in conditioned environment at room temperature of 24-26°C and constant humidity of 38-40%. Patients with atopic dermatitis (60 patients per each experimental group) were applied with 4 different type of creams (experimental group 1, 2, 3, 4) and control cream formulation (control group 1 and 2) as shown in Table 4, on their whole body one month to assess the improvement of atophy. The assessment was carried out by randomly selecting six surrounding areas where the arm folds and measuring the level of erythema diminished by CR-300 Chroma Meter and also grading the level of irritation through patient questionnaire. The two measurements were averaged and the mean value in scales represented the level of skin improvement.

The relative improvement level was compared according to the following evaluation criteria, 0 representing no change in improvement and scales 1 to 5 representing improvements. High numbers indicate bigger improvement in erythema and the skin irritation. Conditions worsened are indicated from -1 to -5. The test result is represented in Table 6 below.

**Table 6**

| Test group | Cream serum-free stem cell culture medium with wheat peptone without peptone (control group 1) | Cream with serum-free stem cell culture medium with wheat peptone pea peptone (experimental group 1) | Cream with serum-free stem cell culture medium with wheat peptone (experimental group 2) |
|---|---|---|---|
| Level of skin improvement | -2.0 ± 0.1 | 3.5 ± 0.3 | 3.8 ± 0.2 |

| Test group | Cream without peptone | Cream with pea peptone | Cream with wheat peptone |
|---|---|---|---|
| | (control 1) | (experimental group 3) | (experimental group 4) |
| Level of skin improvement | -2.7 ± 0.4 | 2.8 ± 0.4 | 3.2 ± 0.2 |

### Example 13: The effect of the topical composition comprising plant peptone or culture medium of stem cells cultured with plant peptone for improving skin condition on anti-aging

The effect of anti-aging in human can be evaluated through several criteria but checking for skin wrinkles are convenient indicator of anti-aging. The effect of the composition comprising plant peptone or culture medium of stem cells (human cord blood-derived mesenchymal stem cells, CB-MSCs) cultured with plant peptone on improving skin wrinkles were tested. The cream prepared in Table 4 was used. The effect on skin improvement was measured by change in skin elasticity. The evaluation of skin elasticity was performed in conditioned environment at room temperatures of 24-26°C and constant humidity of 38-40%. Twenty healthy females (ages 25 to 35) were applied with 4 different types of creams (experimental group 1, 2, 3, 4) and control cream formulation (control group 1 and 2) as shown in Table 4. Skin elasticity was measured with Cutometer SEM 474 (Courage Kazaka, Colongne, Germany). The evaluation criteria were scaled from 0 to 5, representing no elasticity to highest skin elasticity was used to compare their relative improvement levels. The test result is represented in Table 7 below.

**Table 7**

| Test group | Cream with serum-free stem cell culture medium without peptone | Cream with serum-free stem cell culture medium with pea peptone | Cream with serum-free stem cell culture medium with wheat peptone |
|---|---|---|---|
| | (control group 1) | (experimental group 1) | (experimental group 2) |
| Skin elasticity | 2.2 ± 0.3 | 2.9 ± 0.4 | 3.3 ± 0.2 |

| Test group | Cream without peptone | Cream with pea peptone | Cream with wheat peptone |
|---|---|---|---|
| | (control 1) | (experimental group 3) | (experimental group 4) |
| Skin elasticity | 2.2 ± 0.3 | 3.0 ± 0.3 | 3.2 ± 0.2 |

As shown in Table 7, this invention provides evidence that peptone containing creams (experimental group 1, 2, 3, 4) had greater effect on improving skin elasticity compared to the control cream formulation (control group 1 and 2). In other words, the cream comprising culture medium of stem cells cultured with peptone (wheat peptone or pea peptone) and peptone (wheat peptone or pea peptone) alone, has effect on improving skin elasticity.

### Example 14: Anti-aging effect by oral administration

Aging is a process occurring in all living organisms over time in every tissues and organs. Skin in animal is an organ with the biggest surface area and forming the outer layer of the individual, therefore, checking for skin wrinkles are convenient indicator of aging. Especially, UV irradiation can artificially induce aging process, therefore, oral administration or topical administration of a compound in animals after UV irradiation can be used to evaluate the effect of a compound in anti-aging.

To study the effect of the composition on photoaging symptoms, hairless mouse was selected as the experimental animal model. Six to seven week old hairless mice (Skh:HR-1) were divided into following groups each consisting of 8 mice; normal group, UV control group, UV/peptone treatment group, and UV/stem cell culture medium treatment group and raised during the experimental period. For normal group and UV control group, 0.5 ml of saline solution was orally administered to the mice. The UV/peptone treatment group and UV/stem cell culture medium treatment group received solid weight of 500 mg/Kg of body weight peptone (pea peptone or wheat peptone) or stem cell (human cord blood-derived mesenchymal stem cell) culture medium mixed with 0.5 ml of saline solution by oral administration using injection syringe for fluids.

The animals were dosed daily at the same time for 5 days a week for and a total oral administration duration of 5 weeks. Two weeks to five weeks after the oral administration, UV control group, UV/peptone treatment group, and UV/stem cell culture medium treatment group were radiated with UV light similar as natural lights, for 3 times a week. The total UV irradiation was 600 mJ/cm² over the period of the experiment. For an objective decision of the effect on improving skin wrinkles, which is an important phenomenon of anti-aging, skin replicas were made from the back skin of the hairless mice using a silicon polymer, and also from the sample treatment group after 5 weeks prior to killing to assess the anti-aging effect, which is improvement of skin wrinkles. As shown in Table 8, R1, R2, R3, R4, R5, which are parameters for the wrinkle improvement were reduced in peptone and stem cell culture medium using serum-free medium with peptone administered hairless mice. The result indicates that the composition in this invention has an effect on improving the skin wrinkles. That is, when orally administered, stem cell culture medium using serum-free medium with peptone, as well as peptone showed an effect on improving skin wrinkles, suggesting the anti-aging effect (Table 8).

**Table 8**

| R-parameter | UV/control group | UV/pea peptone | UV/wheat peptone | UV/ serum-free stem cell culture medium with wheat peptone | UV/ serum-free stem cell culture medium with pea peptone |
|---|---|---|---|---|---|
| R1 value | 0.52+0.0186 | 0.45+0.00172 | 0.44+0.0163 | 0.46+0.0032 | 0.45+0.0045 |
| R2 value | 0.38+0.0084 | 0.31+0.0088 | 0.30+0.0097 | 0.32+0.0057 | 0.32+0.0065 |
| R3 value | 0.26+0.0061 | 0.21+0.0076 | 0.21+0.0045 | 0.22+0.0088 | 0.23+0.0032 |
| R4 value | 0.12+0.0057 | 0.04+0.0045 | 0.03+0.0023 | 0.03+0.0049 | 0.03+0.0021 |
| R5 value | 0.28+0.0082 | 0.15+ 0.0065 | 0.13+0.0038 | 0.14+0.0056 | 0.15+0.0059 |

R1 value: Skin roughness, R2 value: Maximum roughness, R3 vlalue: Average roughness, R4 value: Smoothness depth, R5 value: Arithmetic average roughness (International Journal of Cosmetic Science, 2005 Jun; 27(3):155-60).

### Example 15: The effect of the melanocyte culture medium differentiated from stem cells cultured in plant peptone and in serum-free medium with plant peptone on improving vitiligo

The effect of the cell culture medium collected after differentiating human cord blood-derived mesenchymal stem cells (CB-MSCs) in culture medium containing plant peptone and plant peptone (wheat peptone or pea peptone) alone on vitiligo were analyzed through clinical tests.

The cell culture medium collected after differentiating cord blood-derived mesenchymal stem cells (CB-MSCs) into melanocytes were prepared according to the following method. Stem cells cultured in normal condition were inoculated and plated at a density of 3 x 10⁵ cells in 6-well plate. The following day, differentiation inducers, stem cell factor (SFC), endothelin-3 (ET-3) and forskolin (FK) were added to the melanocyte culture medium M254 then cultured for 30 days. Thirty days later, the shape of human cord blood-derived mesenchymal stem cell (CB-MSCs) differentiated into melanine cell-like shapes were confirmed. The increase in the gene expression of melanocyte-spedfic MITF-M isoform was confirmed by real-time PCR method. The culture medium was collected and filtered through 0.2 µm micro syringe filter to remove cells before using the medium.

The cream containing 1% each of cell culture medium from cord blood-derived mesenchymal stem cells (CB-MSCs) differentiated into melanocytes or wheat peptone or pea peptone were applied on one hand of the vitiligo test subject. The cream depleted of cell culture medium from cord blood-derived mesenchymal stem cells (CB-MSCs) differentiated into melanocytes or cream without peptone/wheat peptone were applied on the other hand. The level of overall repigmentation was observed at 2-weekly intervals for 3 months. After 3 months of treatment, there was an improvement in patients applied with cream containing culture medium from cord blood-derived mesenchymal stem cells (CB-MSCs) differentiated into melanocytes or cream containing wheat peptone or pea peptone.

The evaluation of vitiligo was performed in conditioned environment at room temperatures of 24-26°C and constant humidity of 38-40%. Patients with vitiligo (40 patients) were applied with 4 different type of creams (experimental group 1, 2, 3, 4) and control cream formulation (control group 1 and 2) as shown in Table 4, on one hand for twice/day for 6 months and observed its effect on improving vitiligo. The evaluation criteria were scaled from 0 to 5, representing no change to significant improvement and their relative values were compared. The test result is represented in Table 9 below.

**Table 9**

| Test group | Cream with serum-free stem cell culture medium (control group 1) | Cream with serum-free stem cell culture medium with pea peptone (experimental group 1) | Cream with serum-free stem cell culture medium with wheat peptone (experimental group 2) |
|---|---|---|---|
| Vitiligo improvement level | 0.0 ± 0.0 | 3.5 ± 0.3 | 3.8 ± 0.2 |
| | | | |

| Test group | Cream without peptone | Cream with pea peptone | Cream with wheat peptone |
|---|---|---|---|
| | (control 1) | (experimental group 3) | (experimental group 4) |
| Vitiligo improvement level | 0.0 ± 0.0 | 2.8 ± 0.4 | 3.2 ± 0.2 |

As shown in Table 9, the cream in this invention had greater effect on improving vitiligo compared to the creams of the control group 1 and 2.

### Example 16: Evaluation of the efficacy on epithelial stem cells from hairless mice epidermis

According to Example 14, the animals were dosed at the same time of the day for 5 days per week for the total duration of 5 weeks. Hairless mice were orally administered with wheat peptone, pea peptone, serum-free stem cell (human cord blood-derived mesenchymal stem cells, CB-MSCs) medium containing wheat peptone or serum-free stem cell (human cord blood-derived mesenchymal stem cells, CB-MSCs) medium containing pea peptone, before isolating epithelial stem cells from epidermis. The epidermal stem cells were isolated according to the methods disclosed above. The isolated epidermal stem cells were inoculated and plated at a density of 3 x 10⁵ cells in 6-well plate. The cells were incubated for 48 hrs in serum containing medium then the cell activity was measured using CCK assay method (cell viability assay method).

**Table 10**

| | UV/control group | UV/pea peptone | UV/wheat peptone | UV/serum-free stem cell culture medium with wheat peptone | UV/serum-free free stem cell culture medium with pea peptone |
|---|---|---|---|---|---|
| Cell increase rate | 100% | 123% | 128% | 134% | 132% |

As shown in Table 10, there was increase in stem cell proliferation rate in experimental groups compared to UV/control group. These results suggest that oral administration of peptone or serum-free stem cell culture medium with peptone have an effect on human stem cell activation.

### Example 17: Acute oral toxicity test

In all of the experimental groups containing wheat peptone, pea peptone, serum-free stem cell (human cord blood-derived mesenchymal stem cells, CB-MSCs) medium containing wheat peptone or serum-free stem cell (human cord blood-derived mesenchymal stem cells, CB-MSCs) medium containing pea peptone, there was an increase of effect on stem cell activation as well as on anti-aging effect when administered topically or orally. Therefore, there is a possibility of commercializing these materials into health foods. Acute oral toxidty test was performed on experimental animals to prevent possible side effects by these materials when used as food ingredients, despite all except for the stem cell culture medium have been approved for human use. Wheat peptone, pea peptone, serum-free stem cell medium containing wheat peptone or serum-free stem cell medium containing pea peptone were tested for acute oral toxicity to check its safety when used as food ingredients. Twenty of 5- to 6-weeks-old SPF Sprague Dawley rats were used for acute toxicity test under following conditions.
- Temperature and humidity conditions : temperature 22 ± 2°C
- Light-dark cycle : fluorescent light lamp (8 am light on, 8 pm light off)
- Illumination level: 200 to 300 Lux
- Free access to UV treated drinking water
- Administration reagent was prepared by diluting the test material in distilled water, for control group, equal amounts of distilled water were administered

On the day of administration, the SPF SD rats were observed every hour for 4 hours after dosing then daily for 14 days, thereafter. The toxicity was determined by carefully observing the behavioral changes, toxic symptoms, mobility, appearance, autonomic nerves, mortality and through evaluating their pathological anatomy. The LD50 of wheat peptone, pea peptone, serum-free stem cell medium containing wheat peptone or serum-free stem cell medium containing pea peptone was 24500, 24300, 24400, 24600mg/kg B.W respectively, and were proved to have no toxicity.

### Example 18: Preparation of health food composition

Since there was no toxicity reported in Example 17 and also showed an effect on anti-aging, these materials were used in developing health foods. The food composition was prepared from each component shown in Table 11, according to the conventional method of preparing a liquid agent. The final volume of each liquid agent is 100 ml. This product example can be applied in manufactured into conventional food forms, such as tablet, powder and granules as well as in liquid forms.

**Table 11**

| component | Amount (wt%) |
|---|---|
| Wheat peptone | 100 mg |
| Pea peptone | |
| Serum-free stem cell culture medium with wheat peptone | |
| Serum-free stem cell culture medium with pea peptone | |
| Honey | 1500 mg |
| Vitamin C | 50 mg |
| Vitamin B₆ | 10 mg |
| Nicotinic add amide | 10 mg |
| Royal jelly | 80 mg |
| Preservative, fragrance, colorant | Trace amount |
| Distilled water | Residual amount |
| **Total** | 100ml |

### Example 19: Effect on increase in energy

Wheat peptone (experimental group 1), pea peptone (experimental group 2), serum-free stem cell (human cord blood-derived mesenchymal stem cells, CB-MSCs) medium containing wheat peptone (experimental group 3), or serum-free stem cell (human cord blood-derived mesenchymal stem cells, CB-MSCs) medium containing pea peptone (experimental group 4) from Example 16 were used to observe the effect on energy increase in hairless mice model. The animals were orally dosed with the experimental composition at the same time of the day for 5 days per week for the total duration of 4 weeks. Five weeks later, animals were sacrificed and immune cells such as SP (splenocytes), PBMC (peripheral blood mononuclear cell), LN (Lymph node) were isolated from the organs. The effect of experimental groups on generating ATP was compared with that of the control group. Each cells were washed twice with PBS the cell lysate was collected. Protein was precipitated by 0.6 M perchloric add and discarded after centrifugation. The supernatant was carefully removed from the protein pellets and were neutralized with KOH. The samples were centrifuged at low speed to remove potassium-percholorate. The supernatant colleted after this step was used for ATP synthesis. ATP synthesis was assessed by the reduction of glucose, hexokinase glucose-6-phosphate dehydrogenase by NADP⁺. Oligomysin was used as an ATPase inhibitor. The result is represented in Table 12 below.

| Content of ATP | SP | PBMC | LN |
|---|---|---|---|
| Oligomysin | 82 ± 4 | 82 ± 4 | 82 ± 4 |
| Control group | 88 ± 3 | 85 ± 4 | 87 ± 5 |
| Experimental group 1 | 105 ± 5 | 98 ± 5 | 102 ± 3 |
| Experimental group 2 | 108 ± 3 | 95 ± 4 | 100 ± 6 |
| Experimental group 3 | 112 ± 6 | 105 ± 6 | 110 ± 6 |
| Experimental group 4 | 11 6 ±6 | 108 ± 6 | 108 ± 6 |

As shown in Table 12, experimental groups 1,2, 3, and 4 had an effect on increasing the energy level in immune cells.

In summery, this invention confirmed that plant peptone can reduce or substitute the use of animal serum when culturing the stem cells. Also, plant peptone were shown to function as an inducer for stem cells. In addition, the stem cell medium removed of stem cells containing plant peptone had an effect on improving the skin condition when administered topically or orally.

Having described a preferred embodiment of the present invention, it is to be understood that variants and modifications thereof falling within the spirit of the invention may become apparent to those skilled in this art, and the scope of this invention is to be determined by appended claims and their equivalents.

## Claims

1. A serum-free medium for culturing a stem cell, comprising a plant peptone as an active ingredient.

2. The serum-free medium according to claim 1, wherein the plant peptone is a product by cleaving a plant-derived protein through acid treatment, base treatment, enzymatic treatment, high-pressure treatment, heat treatment or physical treatment.

3. The serum-free medium according to claim 1, wherein the plant peptone is soy peptone, wheat peptone, broadbean peptone, potato peptone, pea peptone, Papaic soy peptone or lupin peptone.

4. A composition for improving skin condition, comprising a plant peptone as an active ingredient

5. The composition according to claim 4, wherein the plant peptone is a product by cleaving a plant-derived protein through add treatment, base treatment, enzymatic treatment, high-pressure treatment, heat treatment or physical treatment.

6. The composition according to claim 4, wherein the plant peptone is soy peptone, wheat peptone, broadbean peptone, potato peptone, pea peptone, Papaic soy peptone or lupin peptone.

7. The composition according to claim 4, wherein the improvement in skin condition is amelioration of inflammatory skin diseases, improvement in skin wrinkle, inhibition of skin aging, improvement in skin elasticity, improvement in vitiligo, wound healing or skin regeneration.

8. The composition according to claim 7, wherein the inflammatory skin disease is atopic dermatitis, eczema, acne or psoriasis.

9. The composition according to claim 4, wherein the composition is a functional food composition.

10. The composition according to claim 9, wherein the functional food composition is in the form of a solution, a suspension, an emulsion, a paste, a gel, a cream, a powder, a bar, a candy, an ice cream, an oil, a powdered emulsion or a spray.

11. The composition according to claim 4, wherein the composition is a cosmetic composition.

12. The composition according to claim 11, wherein the cosmetic composition is in the form of a solution, a suspension, an emulsion, a paste, a gel, a cream, a lotion, a shampoo, a powder, a soap, a surfactant-containing cleanser, an oil, a powder foundation, an emulsion foundation, a wax foundation or a spray.

13. The composition according to claim 4, wherein the plant peptone promotes proliferation of stem cells or activates stem cells.

14. The composition according to claim 13, wherein the stem cells are adult stem cells in skin.

15. The composition according to claim 13, wherein the plant peptone is contained in the amount of 0.01-30 wt%.

16. A composition for improving skin condition, comprising a serum-free stem cell medium removed of stem cells as an active ingredient wherein the serum-free medium removed of stem cells is prepared by culturing stem cells in a serum-free medium and removing stem cells from the serum-free medium.

17. The composition according to claim 16, wherein the plant peptone is a product by cleaving a plant-derived protein through acid treatment, base treatment, enzymatic treatment, high-pressure treatment, heat treatment or physical treatment.

18. The composition according to claim 16, wherein the plant peptone is soy peptone, wheat peptone, broadbean peptone, potato peptone, pea peptone, Papaic soy peptone or lupin peptone.

19. The composition according to claim 16, wherein the plant peptone in the serum-free medium is contained in the amount of 0.1-10%(g/ml).

20. The composition according to claim 16, wherein the stem cells are human cord blood-derived mesenchymal stem cells, skin-derived stem cells or adipose-derived stem cells.

21. The composition according to claim 16, wherein the improvement in skin condition is amelioration of inflammatory skin diseases, improvement in skin wrinkle, inhibition of skin aging, improvement in skin elasticity, improvement in vitiligo, wound healing or skin regeneration.

22. The composition according to claim 21, wherein the inflammatory skin disease is atopic dermatitis, eczema, acne or psoriasis.

23. The composition according to claim 16, wherein the composition is a functional food composition.

24. The composition according to claim 23, wherein the functional food composition is in the form of a solution, a suspension, an emulsion, a paste, a gel, a cream, a powder, a bar, a candy, an ice cream, an oil, a powdered emulsion or a spray.

25. The composition according to claim 16, wherein the composition is a cosmetic composition.

26. The composition according to claim 25, wherein the cosmetic composition is in the form of a solution, a suspension, an emulsion, a paste, a gel, a cream, a lotion, a shampoo, a powder, a soap, a surfactant-containing cleanser, an oil, a powder foundation, an emulsion foundation, a wax foundation or a spray.

27. The composition according to claim 16, wherein the composition promotes proliferation of stem cells or activates stem cells.

28. The composition according to claim 16, wherein the stem cells are adult stem cells in skin.

29. The composition according to claim 16, wherein the serum-free stem cell medium removed of stem cells is contained in the amount of 0.01-30 wt%.
